# EUROPEAN PATENT SPECIFICATION

(11) **EP 2 433 127 B1**
(45) Date of publication and mention of the grant of the patent: **02.04.2014**
(21) Application number: 10777281.6
(22) Date of filing: 21.05.2010
(51) Int. Cl.: G01N 33/52, A01K 1/015

(54) **COMPOSITE ANIMAL LITTER MATERIAL AND METHODS**
VERBUNDTIERSTREUMATERIAL UND VERFAHREN DAFÜR
MATÉRIAU COMPOSITE POUR LITIÈRE POUR ANIMAUX, ET PROCÉDÉS CORRESPONDANTS

(30) Priority: 22.05.2009 US 180635 P
(43) Date of publication of application: 28.03.2012
(73) Proprietor: 7905122 Canada Inc., Boucherville QC J4B 2X6 (CA)
(72) Inventor: JOLLEZ, Paul, Sherbrooke, Québec J1J 1G9 (CA); BOLDUC, Isabelle, Saint- Hubert, Québec J3Y 1Z5 (CA); BERRADA, Mohammed, Longueuil, Québec J4M 2M5 (CA)
(74) Representative: Mena, Sandra
(86) International application number: PCT/CA2010/000789
(87) International publication number: WO 2010/133001

(56) References cited:
- WO-A1-98/58533
- GB-A- 1 240 884
- US-A- 5 735 232
- US-A- 6 039 004
- US-A1- 2003 170 905
- MCGLASHAN, S.A. ET AL.: 'Preparation and characterisation of biodegradable starch-based nanocomposite materials' POLYM. INT. vol. 52, 2003, pages 1767 - 1773, XP008148162

## Description

### FIELD OF THE INVENTION

This invention generally relates to the field of domestic animal disease detection and more particularly to composite animal litter materials and methods.

### BACKGROUND OF THE INVENTION

Domestic animals such as cats are susceptible to various diseases, ailments and conditions, which are not only arduous and painful for the animal itself but also a source of concern and stress for animal owners. While animal owners nurture, watch over and bestow affection on their pets, they must balance this attention with other responsibilities. Convenience is thus an important factor when taking care of a domestic animal. While owners may be devoted and considerate to their pets, they may lack the sophistication to diagnose animal diseases, ailments and conditions. Convenient, simple and effective means to inform pet owners of the presence of diseases, such as urinary infections, are desired so that appropriate steps can be taken to reverse, mitigate or avoid serious illness in the animal.

For example, feline urinary tract disease can be a serious condition for cats. In feline urinary tract disease, crystals of magnesium ammonium phosphate can precipitate in the cat's urinary tract and cause obstruction. If untreated, the obstruction can lead to intense pain and can often be fatal within days. In some cases, upon observing feline urinary tract disease symptoms - such as bloody urine and urination discomfort and straining - cat owners often consult their veterinarian who may be able to provide treatments, which may be expensive. However, many cats with feline urinary tract disease do not show any obvious symptoms, which is why this disease has been referred to as a "silent killer".

Early detection of feline urinary tract disease is therefore of paramount importance in facilitating treatment, lessening the likelihood of severe complications or aggravations, and reducing the cost of treatment.

Some methods of early detection are known. Early detection may be possible by occult blood testing, allowing cat owners to treat the problem of feline urinary tract disease by changing the cats' diets. However, some known occult blood testing techniques present various disadvantages concerning the complexity and inconvenience of the tests. For instance, cats are creatures of routine and will often resist urine sample gathering.

Also known are some detection agents that may be combined with animal litter or used as animal litter to allow daily assessment of the animal's health.

Diagnostic beads comprising a particulate material and a detection composition, either coated on the surface or impregnated into the particulate material, for the detection of occult blood in animal excreta have been described in US 2003/0170905. These diagnostic beads are part of the animal litter itself and not require any additional vehicle for carrying out the diagnostic test. The detection composition comprises a chromogen such as 3,3',5,5' -tetramethyl benzidine which produces a visible and immediate color change when excreta containing blood comes into contact with the diagnostic beads, a peroxide stable for prolonged periods of time such as cumene hydroperoxide, an enhancer such as 6-methoxyquinoline, a stabilizer such as ascorbic acid and a binder that is a starch derivative or a polymeric material capable of adhering onto the diagnostic beads.

It is known to use diagnostic agents, incorporated into test strips, beads or particles, for detection purposes. Usually, such test strips consist of an absorbent carrier made from fibrous or non-woven material, in the simplest case filter paper, which is coated or impregnated with the detection reagents. Components of the detection reagent may be a chromogen as an indicator, an oxidizing agent such as a hydroperoxide as an activator of the indicator. The oxidizing agent, which is sometimes also called a sensitizer or an accelerator, increases the sensitivity of detection. Standard additional components are, apart from a surface-active agent (wetting agent), thickening agents which prevent the bleeding of the wetted test field, pigments, complex-forming agents and/or other stabilizers for the chromogen and/or the hydroperoxide.

Similarly, various analytical methods are presently available for detecting the presence of "peroxidatively active substances" in samples such as urine, fecal suspensions, and gastrointestinal contents. Hemoglobin and its derivatives are typical of such peroxidatively active substances because they behave in a manner similar to the enzyme peroxidase. Such substances are also referred to herein as pseudoperoxidases. Peroxidatively active substances are enzyme-like in that they catalyze the redox reaction between peroxides and benzidine, o-tolidine, 3,3',5,5'-tetramethylbenzidine, 2,7-diaminofluorene or similar benzidine-type indicator substances, thereby producing a detectable response such as a color change. Most methods for determining the presence of occult blood in test samples rely on this pseudoperoxidase activity.

A benzidine-type indicator responds in the presence of hydrogen peroxide and peroxidase by changing its light absorptive capability.

Providing a reliable occult blood detection system in animal litter itself also has many problems and challenges. For example, the test indicator material should be stable when exposed to a wide variety of ambient conditions, be they dry or humid, and over a wide range of temperatures. Such stability is quite often difficult to achieve.

A further problem with many known test indicators is that pet owners are insufficiently observant or sophisticated to appreciate the positive indication, such as a color change, before the indicator decays. Many known indicators do not stay at the changed color for a sufficient period of time to allow pet owners to reliably recognize the indicated health issue.

An additional problem with various detection reagents mixed with animal litter is that the test reagents give off sufficient scent such that cats, which have an extraordinary sense of smell, recognize the odor change in their litter and thus tend to shy away from the litter. As will be appreciated, this not only defeats the purpose of a convenient detector but can also cause unwanted excretory mishaps. Thus, test reagents with significant, offensive or upsetting odors - both to the user and the cat - have many disadvantages.

A further problem with known detection reagents is poor shelf life stability, particularly if combined with an animal litter for storage as a single mixture. Poor stability leads to disadvantages in the ability to store, transport, display, purchase and use the detection-litter combination.

Detection materials that are merely coated over the surface of a carrier material also have various disadvantages that may relate to poor shelf-life stability, low in-use stability and lifetime, and insufficient color change visibility.

Some other animal litters or detection additives are difficult or complicated to manufacture requiring multiple steps of impregnation, drying, coating, material cutting, etc.

Known materials and methods for detection of feline urinary tract disease have involved one or more of the above deficiencies. There is a need for a technology that overcomes at least some of these deficiencies.

Clays and other mineral compositions such as diatomaceous earth are environmentally friendly, naturally abundant and economic. Even though many types of clay are known for their liquid absorbing properties, their use is often restricted due to their colloidal, dispersive properties in water. The use of clays in combination with other ingredients such as polymers is known.

Nanocomposites can be prepared by numerous techniques. The most common technique involves ion exchange of the cations located in the interlayer spacing of the clays using cationic surfactants (cationic molecules bearing C₈ - C₃₀ aliphatic chains). This technique was first reported by Okada et al. (Mat. Res. Soc. Proc., 1990, 171, 45-50) and subsequently by Pinnavaia et al. (U.S. Pat. No. 6,261,640; U.S. Pat. No. 6,414,069, and U.S. Pat. No. 6,261,640).

Techniques for increasing the interlayer spacing between the phyllosilicates making up the clays have been described by Beall et al. (U.S. Pat. No. 6,228,903 and U.S. Pat. No. 5,760,121); Lan et al. (U.S. Pat. No. 6,399,690); Qian et al. (U.S. Pat. No. 6,407,155); Zilg et al. (U.S. Pat. No. 6,197,849), Ross et al. (U.S. Pat. No. 6,521,690); Barbee et al. (US20020169246 A1); Ishida (U.S. Pat. No. 6,271,297); Powell et al. (U.S. Pat. No. 6,730,719); Knudson et al. (US20020165305 A1); Lorah et al. (US20030060555 A1); Fischer et al. (U.S. Pat. No. 6,579,927) and Bagrodia et al. (U.S. Pat. No. 6,586,500).

Nanocomposites have also been prepared using physico-chemical techniques such as extrusion, lyopfiilization, and ultrasonic wave treatments, as disclosed by Torkelson et al. (WO 2004043663); Lee et al. (US20030134942 A1); Nobuyoshi (JP 02-203936), and McClelland et al. (CA 2,352,502).

Hybrid organic-inorganic gels for use in cosmetic or pharmaceutical compositions have been disclosed by Lahanas et al. (U.S. Pat. No. 6,042,839); Udagawa (JP 09-187493); Collin et al. (EP 1327435 A1); and Chevalier et al. (EP 1203789 A1). However, these gels have not been reported as detectors for feline urinary tract disease applications.

Starches have also been reported as being used as components in nanocomposite materials. Hydroxyapatite reinforced starch/ethylene-vinyl alcohol copolymer composites have been reported by Reis et al. (J. Adv. Polym. Technol. 1997, 16, 263). Calcined kaolin/thermoplastic starch composites have been disclosed by DeCarvalho et al. (Carbohydr. Polym. 2001, 45 (2), 189-194). Montmorillonite/thermoplastic starch hybrids have been described by Park et al. (Macromolecular Materials and Engineering, 2002, 287(8), pp. 553-558, J. of Mat. Sci, 2003, 38 (5), pp. 909-915) and McGlashan et al. (Polymer International, 2003, 52(11), PP 1767-1773). However, these starch containing nanocomposite materials were not reported as exhibiting feline urinary tract disease detection properties.

The use of chitosan in nanocomposite materials has also been reported. Cationic chitosan, intercalated in montmorillonite, has been disclosed by Darder et al. (Chemistry of materials, 2003, 15 (20), PP 3774-3780). A butyl-acrylate-graft chitosan montmorillonite nanocomposite, has been reported by Li et al. (Radiation physics and chemistry, 2004, 69(6) APR, PP 467-471). The use of xanthan and scleroglucan in nanocomposite materials has also been reported.

Takahiro et al (Japanese Patent No. 01-296933), Marx (U.S. Pat. No. 4,615,923) and Brander et al (U.S. Pat. No. 6,376,034) describe inorganic additives (kieselguhr, clays, diatomaceous earth) added to biodegradable superabsorbents. However, none of these patents teach additives made from organic components.

In summary, there is a need for a technology that provides animal litter that enables the detection of conditions such as feline urinary tract disease and overcomes at least some of the drawbacks of what is already known.

### SUMMARY OF THE INVENTION

The present invention responds to the above need by providing a composite material allowing detection of certain conditions.

In one aspect of the present invention, there is provided a composite litter material for detecting a disease when contacted by feline urine, the composite litter material comprising: an absorptive polymer material forming a solid matrix; exfoliated clay embedded and dispersed within the solid matrix; a chromogenic indicator provided within the solid matrix; and an oxidizing agent distributed and stabilized within the solid matrix such that the oxidizing agent is available and responsive to peroxidase or pseudoperoxidase activity in the urine to activate the chromogenic indicator.

in an optional aspect, the absorptive polymer material is a polysaccharide. In another optional aspect, the polysaccharide comprises alpha-linkages. In another optional aspect, the exfoliated clay comprises exfoliated phyllosilicates. In another optional aspect, the exfoliated phyllosilicates are dispersed throughout the entire solid matrix, In another optional aspect, the chromogenic indicator is cationic and the exfoliated clay comprises sheets having anionic surface characteristics. In another optional aspect, the chromogenic indicator is a benzidine-based compound. In another optional aspect, the chromogenic indicator corresponds to the following formula: 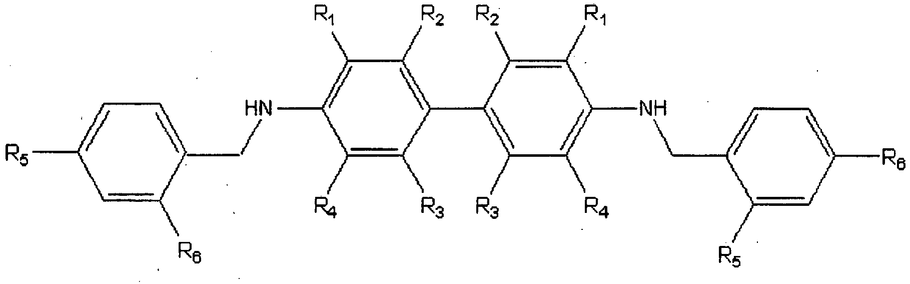 wherein R1, R2, R3 and R4 are the same or different and represent hydrogen, halogen, a lower alkyl or alkoxy group containing 1 to 4 carbon atoms, a (C1 - C4)-dialkylamino group, an acetylamino group, a nitro group or an aromatic group which may be substituted; and
wherein R5 and R6 are the same or different and represent water-soluble groups, hydroxyl group, amino group, acidic group, disulfonyl group, ether group, halogen, and a lower alkyl or alkoxy group containing 1 to 4 carbon atoms, a (C1 -C4)-dialkylamino group, an acetylamino group or a nitro group.

In another optional aspect, the chromogenic indicator is 3,3',5,5'-tetramethylbenzidine (TMB). In another optional aspect, the chromogenic indicator is 3,3',5,5' tetramethylbenzidine dihydrochloride (TMB-HC).

In another optional aspect, the oxidizing agent is a hydroperoxide. In another optional aspect, the oxidizing agent is cumene hydroperoxide. In another optional aspect, the oxidizing agent is diisopropylbenzene dihydroperoxide.

In another optional aspect, the absorptive polymer material comprises polyols having polymer backbones and the hydroperoxide is stabilized along the polymer backbones.

In another optional aspect, the composite material is provided in the form of discrete pellets. In another optional aspect, each pellet has a disk shape, a lozenge shape, a wafer shape or a cat head shape. In another optional aspect, each pellet has a diameter between about 1 mm to about 10 mm and a thickness between about 0.5 mm and about 3 mm.

In another optional aspect, the composite material further comprises an impregnated stabilizer composition. In another optional aspect, the stabilizer composition comprises a color enhancer.

In another aspect of the present invention, there is provided a cat litter for detecting a feline disease when contacted by feline urine, the cat litter comprising: a particulate filler material; composite pellets mixable with the particulate filler material, each of the composite pellets comprising:an absorptive polymer material forming a solid matrix; exfoliated clay embedded and dispersed within the solid matrix; a chromogenic indicator provided within the solid matrix; and an oxidizing agent distributed and stabilized within the solid matrix such that the oxidizing agent is available and responsive to peroxidase or pseudoperoxidase activity in the urine to activate the chromogenic indicator.

In an optional aspect, the particulate litter material comprises clumping or non-clumping clay. In another optional aspect, the particulate litter material and the composite pellets are sized and mixed to as to form a substantially homogeneous mixture.

In yet another aspect of the present invention, there is provided a method for detecting or diagnosing a feline disease, the method comprising:
allowing urine to contact a composite material comprising an absorptive polymer material forming a solid matrix, exfoliated clay embedded and dispersed within the solid matrix, a chromogenic indicator provided within the solid matrix and
an oxidizing agent distributed and stabilized within the solid matrix such that the oxidizing agent is available and responsive to peroxidase or pseudoperoxidase activity in the urine to activate the chromogenic indicator;
detecting or diagnosing the feline disease by determining whether the chromogenic indicator has been activated as evidenced by a color change.

The method may further comprise providing the composite material pre-mixed with a conventional cat litter material and allowing a cat to urinate thereon.

The method may further comprise providing the composite material as part of a testing kit.

In still another aspect of the present invention, there is provided a method of manufacturing a composite animal litter material for detecting a feline disease, comprising:
preparing a mixture comprising an absorptive polymer and water;
distributing exfoliated clay, a chromogenic indicator and an oxidizing agent to within the mixture to produce the composite animal litter material.

In an optional aspect of the method, a solution comprising the exfoliated clay is added to the mixture during the preparation thereof.

In another optional aspect of the method, a chromogenic solution comprising the chromogenic indicator, a solvent and the oxidizing agent is prepared and introduced into the mixture.

In another optional aspect of the method, the chromogenic solution and the mixture are combined by extrusion, pressure agglomeration, tumble growth agglomeration or matrix melt formation, or a combination thereof.

In another optional aspect of the method, the chromogenic solution is buffered at a pH between about 1.5 and about 7. The chromogenic solution may also be buffered at a pH allowing solubility in the absence of alcohol.

In another optional aspect of the method, the chromogenic solution and the mixture are combined by extrusion. The method may comprise: providing an extrusion apparatus having an upstream section, a mid section and a downstream section; feeding the mixture into the upstream section of the extrusion apparatus; feeding the chromogenic solution into the mixture at the downstream section of the extrusion apparatus, to form a combined blend; extruding, cutting and drying the combined blend to form the composite material.

In another optional aspect of the method, the mid section is operated at a higher temperature than the upstream and downstream sections.

In another optional aspect of the method, the temperature of the mid section is about 60 to about 80°C higher than either one of the upstream or downstream sections; optionally, the upstream section is operated at a temperature between about 25 °C and about 40°C, the mid section is operated at a temperature between about 80°C and about 130°C, and the downstream section is operated at a temperature between about 25 °C and about 40°C; and optionally, the upstream section is operated at a temperature of about 30°C, the mid section is operated at a temperature of about 110 °C, and the downstream section is operated at a temperature of about 40°C.

In another optional aspect of the method, it further comprises homogenizing the combined blend. Thus can be done by extending the downstream section or adding another extruder. In another optional aspect of the method, the extrusion apparatus comprises a twin screw extruder. The extrusion apparatus may include a first extruder having an inlet and an outlet, and a second extruder coupled to the first extruder to receive the combined blend from the outlet of the first extruder. The first extruder may be a twin-screw extruder. The second extruder may be a single screw extruder.

In another optional aspect of the method, the pH of the chromogenic solution is acidic. In another optional aspect of the method, the chromogenic solution further comprises an amount of alcohol sufficient for solubilization. In another optional aspect of the method, the exfoliated clay is exfoliated bentonite and is added at less than 1 % w/w with respect to the mixture. In another optional aspect of the method, the absorptive polymer is wheat starch or corn starch. In another optional aspect of the method, the combined blend, after exiting the extruder, is dried at about 50°C.

Further aspects, embodiments and advantages of the present invention will be further understood upon reading of detailed description and the appended drawings.

### BRIEF DESCRIPTION OF THE DRAWINGS

Fig 1 is a cut view schematic representation of a composite material pellet according to an embodiment of the present invention.

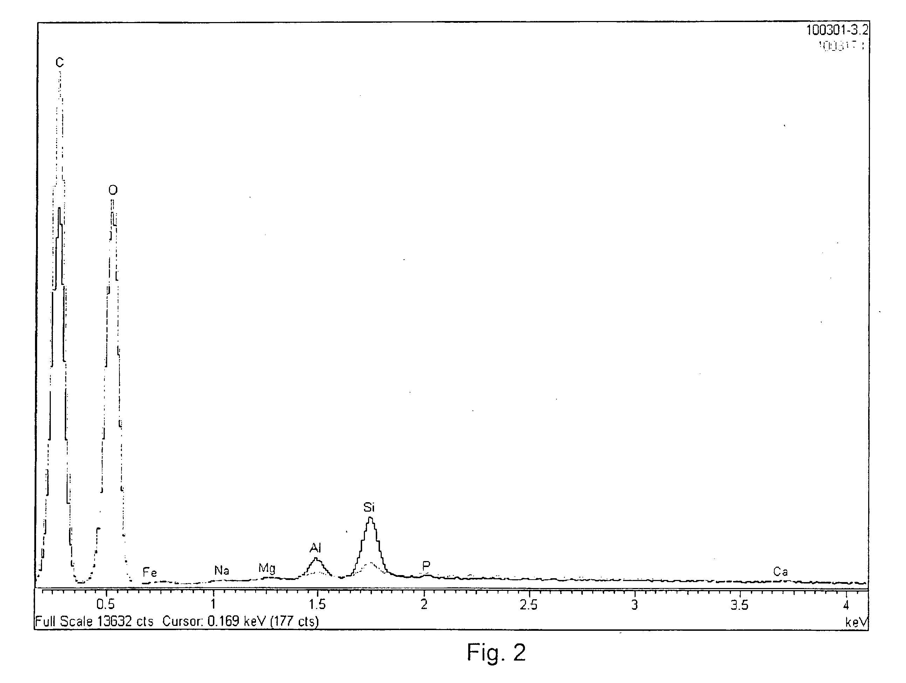

Fig 2 is a graph showing the elementary analysis of a granule of composite material containing wheat starch and exfoliated bentonite by Energy-dispersive X-ray spectroscopy.
Fig 3 is a graph of absorption of pellets versus soaking time.

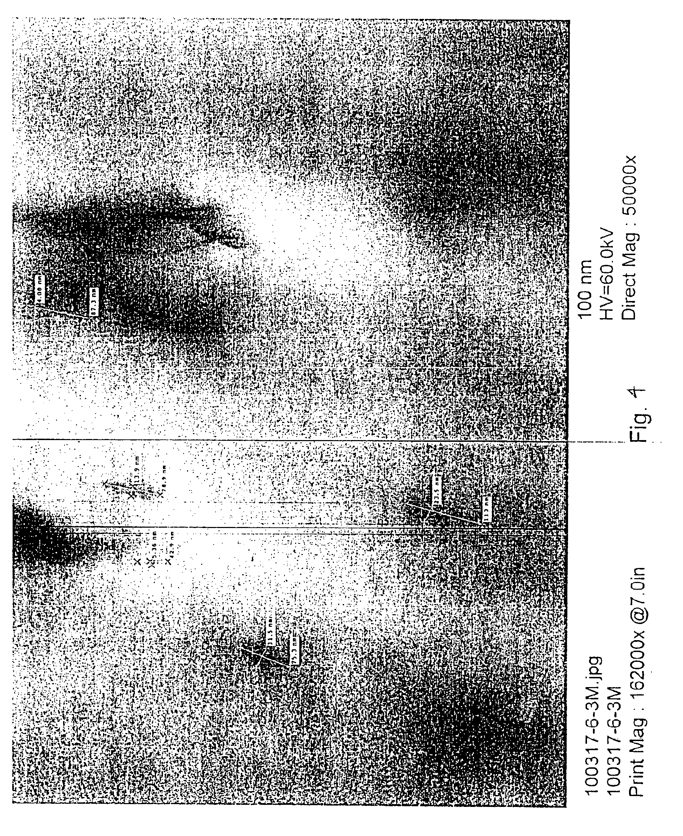

Fig 4 is a photograph MET of wheat starch with 1% exfoliated bentonite.

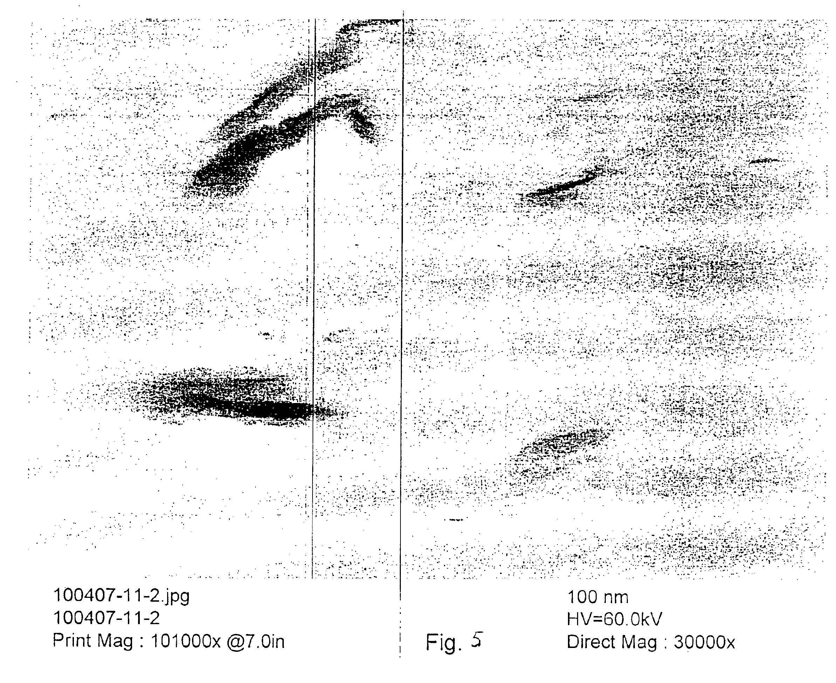

Fig 5 is a photograph MET of wheat starch with 1% exfoliated bentonite (small particles) and chromogenic compound (large particles).

While the invention will be described in conjunction with example embodiments, it will be understood that it is not intended to limit the scope of the invention to such embodiments. On the contrary, it is intended to cover all alternatives, modifications and equivalents as may be included as described by the present description and appended claims.

### DETAILLED DESCRIPTION

The present invention provides a composite material for use in connection with animal litter and having a structure for detection of certain abnormalities in excretions and aqueous solutions such as urine.

Some preferred embodiments described herein pertain to the composite material's use in connection with detecting feline urinary tract disease from peroxidase or pseudoperoxidase activity in feline urine, i.e. in peroxidatic assays. However, it should be understood that the composite material may be used in other applications for detection purposes. For instance, in some embodiments, the composite material is used in connection with detecting elevated glucose levels which can be useful in diagnosing diabetes, via a glucose oxidase-peroxidase system. In this regard, in the presence of glucose oxydase (Aspergillus niger), glucose in aqueous solution is oxidized into gluconic acid by dissolved oxygen with the formation of hydrogen peroxide, which is then dosed by an indicating enzymatic reaction (Horseradish) which oxidizes the chromogen which changes color.

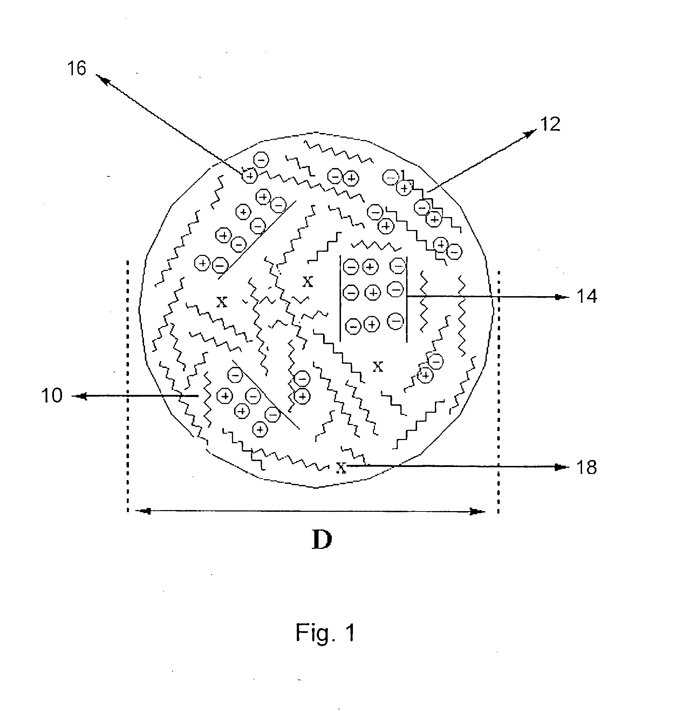

In one embodiment of the present invention, as illustrated in Figure 1, the composite material is provided in the general form of a pellet. By providing a plurality of discrete pellets, the composite material may be easily packaged and may also be pre-mixed with conventional animal litter.

Referring to Figure 1, each composite pellet 10 comprises an absorptive polymer material 12 forming a solid matrix; exfoliated clay 14 embedded and dispersed within the solid matrix; a chromogenic indicator 16 provided in the matrix; and an oxidizing agent 18 distributed and stabilized within the solid matrix such that the oxidizing agent 18 is available and responsive to peroxidase or pseudoperoxidase activity in the feline urine to activate the chromogenic indicator 16.The composite material may also be referred to herein as a "nanocomposite material".

The absorptive polymer material 12, which forms a solid matrix, preferably comprises a polysaccharide, which provides polysaccharide chain backbones in addition to the general polysaccharide matrix. More particularly, polysaccharides that are suitable for use in the nanocomposite material may be selected from the following non-limited group: starches, modified starches, amylopectin, modified amylopectin, amylose, modified amylose and mixture thereof. Amongst these polysaccharides, starch is frequently chosen as a polysaccharide for use in the agglomerated particle. Non-limiting examples of such starches are starch granules, pregelatinized starches, glass-like starches, waxy starches, anionic starches, cationic starches, fractionated starches, cross-linked starches, hydroxyalkylated starches, alkylated starches and mixture thereof. Starch that is suitable for the present invention may be obtained from many sources, including but not limited to wheat, maize, buckwheat, potato, cassaya, sorghum, millet, oat, arrowroot, barley, beans, peas, rice, rye, waxy starches and mixture thereof. A commonly used starch is wheat starch. Naturally occurring starch is usually organized in a semicrystalline, water insoluble pattern, which is sometimes referred to as a "starch granule". The form of these starch granules is characteristic of their botanical origin, and their mean particle size may range from about 1 µm to about 60 µm.

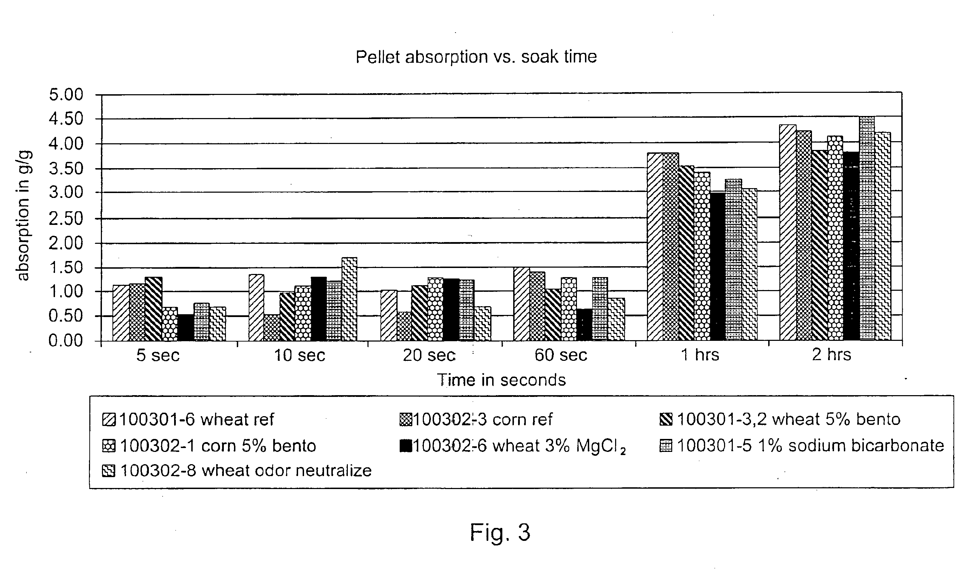

The polymer material 12 absorbs urine with which it comes into contact. The specific polymer and its modification and form may be selected to allow sufficient internal diffusion of the urine on contact and sufficient urine retention to facilitate the chromogenic response over time. The exemplary absorption properties shown in Fig 3 were sufficient for the present invention, though many other absorption rates may also be used.

The exfoliated clay 14 is dispersed throughout the solid polymer matrix. It should be noted that at least a portion of the clay incorporated into the composite material is exfoliated and thus it is possible to include some non-exfoliated clay as well. It should also be understood that the "exfoliated clay" may include fully exfoliated, partially exfoliated, and/or semi-exfoliated clays. Preferably, all of the clay within the composite material is substantially exfoliated clay, dispersed throughout the composite material. Depending on the quatity of exfoliated clay and the characteristics of other compounds included in the composite material, the exfoliated clay may promote binding with the chromogenic indicator 16 and may improve dispersion of the exfoliated clay 14 and the chromogenic indicator 16 throughout the composite material in an efficient and substantially homogeneous manner. Improved dispersion of the chromogenic indicator leads to improved detection functionality of the composite material and homogeneity of the material in general leads to improved structural properties of the composite material. In one optional aspect, the exfoliated clay may be selected and prepared in order to have anionic surface characteristics, the chromogenic indicator is selected to be cationic, and the exfoliated clay and chromogenic indicator are proportioned such that within the solid matrix they may ionically bond.

In one preferred aspect, the clay is a crystalline alkali metal phyllosilicate which is exfoliated. The clay source can be either natural or synthetic. Examples of clays include, but are not limited to smectites, hectorites, bentonites, montmorillonites, Laponites™, celites, illites and mixtures thereof. One preferred clay is bentonite, which is a montmorillonite type clay. Bentonite is essentially made from colloidal hydrated aluminum phyllosilicates and contains varying amounts of iron, alkali, and alkaline earth metals. Calcium, sodium, potassium and magnesium bentonites are preferred. Ion-exchanged benionites can also be used in the preparation of polysaccharide-phyllosilicate composite materials according to embodiments of the present invention.

The composite material of the present invention comprises an inorganic portion that includes exfoliated clay. The inorganic portion may also include other mineral components. The inorganic portion may interact physicochemically with the chromogen. The typical inorganic portion content ranges up to about 40%. The particle size of the inert inorganic portion may range from about 10 µm to about 150 µm. Water adsorbents, such as molecular sieves, zeolites, clays, silicates, silica gel, insoluble salts and mixture thereof may preferably be used. Among this class, swelling clays may also be used. Non limiting examples of inorganic substances are calcium sulfate, silica gel, zeolites and mixtures thereof. Clinoptilolite is a good source of zeolites. Non-limiting examples of swelling clays are smectites, hectorites, bentonites, montmorillonites, Laponites™, celites, illites and mixture thereof. Bentonite has been found to be quite suitable.

The chromogenic indicator 16 allows the oxidizing agent 18 to activate it when the oxidizing agent 18 is triggered by the presence of peroxidase or pseudoperoxidase activity. In one preferred embodiment, the chromogenic indicator 16 is an electron donor, i.e. a reducing agent that changes color upon losing an electron.

In an optional aspect, the chromogenic indicator 16 is homogeneously dispersed throughout the solid polymer matrix, assisted by the preparation method and the presence of exfoliated clay. Thus, the chromogenic indicator is present not only at the exterior surface of a given composite pellet, but also in a neighboring sub-surface region that can be rapidly exposed to urine that absorbs into the pellet. Especially when the solid polymer matrix is glassy or substantially transparent, the presence of the chromogenic indicator in a sub-surface region allows it to be readily visible when color change occurs and also avoids exposure to the air. In some aspects, the chromogenic indicator may also be bound with the exfoliated clay 14 by being adsorbed on the exfolitated clay sheets in the polysaccharide solid matrix. The chromogenic indicator 16 may be intercalated between the exfoliated clay 14.

In one preferred embodiment of the composite pellet 10, the chromogenic indicator 16 may be a compound as shown in Formula I:

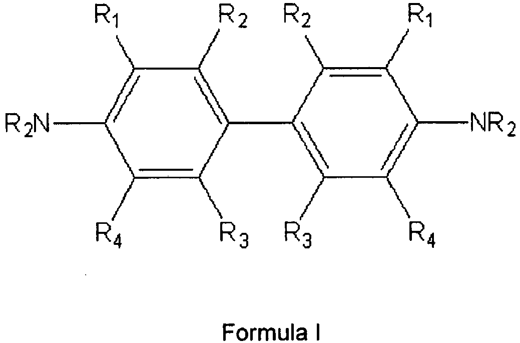

In Formula 2A, R1, R2, R3 and R4 may be the same or different and may be hydrogen, halogen, a lower alkyl or alkoxy group containing 1 to 4 carbon atoms, a (C1 -C4)-dialkylamino group, an acetylamino group, a nitro group or an aromatic group which may be substituted. In one preferred embodiment, the chromogenic indicator 16 may be a compound as shown in Formula II:

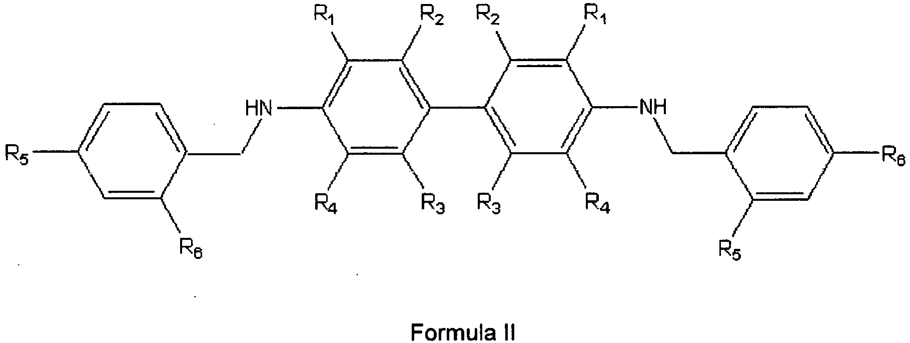

In Formula II, R1, R2, R3 and R4 may be the same or different and represent hydrogen, halogen, and a lower alkyl or alkoxy group containing 1 to 4 carbon atoms, a (C1 -C4)-dialkylamino group, an acetylamino group, a nitro group or an aromatic group which may be substituted; R5 and R6 are the same or different and represent water-soluble groups as hydroxyl groupl, amino group, acidic group, disulfonyl group, ether group, halogen, and a lower alkyl or alkoxy group containing 1 to 4 carbon atoms, a (C1 -C4)-dialkylamino group, an acetylamino group or a nitro group.

Thus, a water soluble benzidine-type indicator of Formula II, responds in the presence of hydrogen peroxide and peroxidese by changing its light absorptive capability, which is due to the chemical transformation to the compound shown in Formula III:

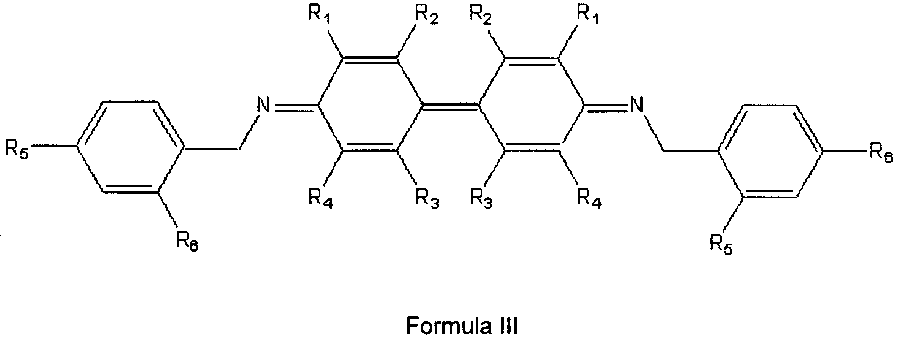

Several different types of benzidene chromogenic indicators 16 may be used in embodiments of the present invention.

The oxidizing agent 18 can be stabilized, by ascorbic acid for example, in the polymer matrix, is reactive to peroxidase or pseudo-peroxidase activity and is able to activate the chromogenic indicator. In one preferred embodiment, the oxidizing agent 18 comprises a hydroperoxide. The hydroperoxide may be, for example, cumene hydroperoxide which is sutable for detection of hemoglobin and also can have some reactivity to elevated glucose levels, thus for urinary tract disease and diabetes. The hydroperoxide may also be, for example, diisopropylbenzene dihydroperoxide which has high selectivity to detection of hemoglobin and thus of urinary tract disease. In some embodiments, both of these hydroperoxides could be used in the same composite material, or in different pellets, the former for more general disease detection and the later for more specific detection of urinary tract disease.

In one embodiment of the present invention, the composite pellet 10 comprises both organic and inorganic components. The composite pellets 10 may indeed be composed of mainly renewable resources and are cost-efficient.

In an optional embodiment of the present invention, the composite pellet 10 may further comprise a surface-active agent, if necessary, other auxiliary agents such as thickeners, stabilizers, pigments, a buffer system and/or complex-forming agents. Each of such additives may be used in pellets for particular applications.

In another optional embodiment of the present invention, the composite material may also comprise a color enhancer. The color enhancer should be chosen depending on the particular components of the composite material, in particular the chromogenic indicator. The color enhancer may comprise 5-isoquinoline sulfonic acid.

The chromogenic indicator and the exfoliated clay particles may be incorporated into the polymer matrix and sized so as to have sizes and distributions as shown in the photographs of the Figs.

The composite pellet 10 is illustrated as having a diameter D which may optionally be from about 1 mm to about 10 mm, and preferably in the range of about 5 mm. The composite pellet 10 may have a thickness between about 0.5 mm and about 3 mm, and preferably in the range of about 1 mm. Each pellet 10 may therefore have a disk shape, a lozenge shape, cat head shape or a wafer shape. This generally flattened shape allows a greater surface area per pellet which leads both to improved absorption of the feline urine and also improved color visibility. The composite pellets 10 are also able to retain the urine better than porous two-dimensional filter paper, surface coatings or non-absorptive beads, for example. It should be understood, however, that the pellets may be produced to have a variety of other shapes and sizes, as desired.

The composite pellets 10 are discrete and may be provided as a separate additive to cat litters, when the detection function is desired. The pellets 10 may also be provided pre-mixed directly with conventional cat litter materials, thus forming part of the packaged litter formula for sale. Conventional cat litters may be clay-based, cellulose-based or another suitable type of animal litter.

The composite pellets 10 of the present invention may be discretely blended with cat litter materials or with other materials or products for animal occult blood detection applications such as various hygiene articles. The composite pellets may be pre-blended with a polymer having absorbance sufficient for a desired purpose or application of a particular composite material. The pellets may also be mixed with fluff pulp and other components in the manufacturing process.

Composite pellets may also be produced with various components in order to detect different feline diseases which are indicated by a predetermined characteristic of the cat's urine such as a particular pH range and/or blood in the cat's urine. Feline diseases which may be thus detected include feline urinary tract diseases, such as feline urological syndrome or feline lower urinary tract disease and/or cystitis (bladder infection).

The embodiments of the present invention have various features and allow several advantages. Composite material embodiments allow accurate testing for feline urinary tract disease and also allow safe, convenient and reliable storage, transportation, and end-use by the pet owner. The composite pellets retain their peroxidatic activity and do not develop a color change during storage. The composite material is also stable when exposed to ambient conditions for several days and provide a positive color response that does not deteriorate significantly for at least 6 hours subsequent to exposure to urine having occult blood. The composite pellets remain stable when exposed to ambient conditions and provide a positive color response that also remains stable for a sufficient time for the color response to be observed. The composite pellets are convenient, stable to substantially avoid spontaneous oxidation, and ready-to-use. The composite pellets can be used as aggregate to be conveniently homogeneously mixed with conventional cat box filler to provide a safe and reliable occult blood test for feline urinary tract disease. The composite material may also be used in various other fields and applications for detection of a condition, disease, ailment, cycle, or generally the presence of a given chemical in a fluid. The composite pellets, alone or in combination with the cat litter, can be produced so as to not have offensive odors that may dissuade the cat from properly using the litter. The pellets can be mixed with conventional cat box filler and still show adequate stability over time despite changing weather or light conditions. The composite material may also be triggered by as few as 100 red blood cells per microliter of cat urine. The pellets may also be produced to have a specific color in their non-reacted state. For instance, the non-reacted color may be chosen to provide various indications, such as an orange color which is found to be the registered trade mark logo color for Le Groupe Intersand Canada Inc. The non-reacted color may also be chosen to obtained better contrast between reacted pellets, non-reacted pellets and the litter material.

In various preparation techniques, separate solutions and mixtures may be prepared and then combined together to produce the end-product composite material.

In one aspect, a first stage of the process includes mixing water and the polysaccharides which then undergo processing to ensure that the solid matrix of the composite material will be "gellified" and avoid breaking or crumbling. The processing of the polysaccharide mixture is usually performed at elevated temperatures, for example around 110°C. The exfoliated clay and the chromogenic indicator may be added to the mixture at various points in processing the polysaccharide mixture. The bentonite is preferably added at the beginning of processing the polysaccharide mixture. The peroxide is preferably added near the end of processing the polysaccharide mixture at a point when the mixture is brought back to a lower temperature, for example around 40°C. This order of processing and addition allows preparation of a gellified, hard, solid solid polymer matrix, while avoiding unwanted pre-mature reaction of the peroxide triggered by elevated temperature, which could lead to deactivation of the end-material and dangerous processing conditions.

In one optional method for manufacturing the composite material, a chromogenic solution is prepared containing a chromogen compound susceptible to peroxidatic oxidation and clay in a suitable solvent that permits the exfoliation of the clay and the high solubility of chromogen under high shear mixing. When the chromogen has high water-solubility, such as TMB hydrochloride, water can be preferably used as the solvent. In addition, a buffered substrate mixture may be separately prepared containing a buffer of pH between about 6 and 8, preferably water or a combination of water with alcohol, a hydroperoxide and a polysaccharide polymer. This mixture is usually highly viscous due to the polysaccharide polymer. Upon mixing of the solution and mixture described above by extrusion or other process to form "nanocomposite particles", the chromogen, oxidizing agent and the exfoliated clay are dispersed throughout the polymer matrix. One difficulty with this technique, however, is that when some compounds (e.g. hydroperoxides) and methods (e.g. extrusion) are used, the desired temperature for processing the polysaccharide mixture can lead to the degradation or reaction of the hydroperoxide. Thus, for such embodiments, the polysaccharide mixture should be handled carefully at reduced temperatures, using stabilizing additives, or choosing oxidizing agents that do not react at the polysaccharide processing conditions, such that the oxidizing agents remain active in the final composite material product.

In another optional method for manufacturing the composite material, which avoids some of the disadvantages mentioned above, a solution is prepared containing a chromogen compound, a solvent and an oxidizing agent. The solution may also include a color enhancer such as lepidine, a buffering compound such as citric acid, a stabilizer such as ascorbic acid, a pH-increasing agent such as sodium hydroxide, surfactants and other additives. The solution may be prepared and tailored to the particular absorptive polymer and processing method to be used. A mixture is also prepared containing an absorptive polymer, a solvent and exfoliated clay. The clay is thus not in contact with the active agents, namely the chromogen and the peroxide as this stage. The solvent for the mixtrue is usually water, in particular when the absorption polymer is a water-soluble polysaccharide such as various starches. The solution and the mixture are then combined together to form the composite material. The solution is preferably added to the mixture once the mixture has undergone heat and shear treatment and has cooled to a sufficiently low temperature such that the chromogen and particularly the oxidizing agent do not degrade or deactivate due to high temperature. In one exemplary method, the mixture is formed in an upstream section of an extruder and the solution is introduced at a point near the exit of the extruder such that the polysaccharide and exfoliated clay mixture have formed a gelled matrix and the components in the solution are quickly dispersed within the gelled matrix prior to exiting the extruder. More regarding this will be explained and understood with reference to the examples.

The solution may be buffered between about 1.5 and about 8 pH. It has been observed that a chromogenic solution having a pH of 1.7 enabled production of reactive composite pellets.

Process conditions may influence morphology and efficiency of the components of the composite pellets. The composite material of the present invention may be formed by pressure agglomeration, tumble growth agglomeration or matrix melt formation.

In order to obtain composite pellets, the polysaccharide and the inorganic exfoliated clay may be uniformly blended together before they will be bound to each other. An agglomerating agent or a binder may optionally be mixed with other components. Non-limiting examples of suitable binders are gelling polysaccharides, such as sodium carboxymethyl cellulose.

Matrix melt formation may be used to form uniform and semi-uniform composites. In matrix melt formation, the polysaccharide component of the composite material may be partially molten (for semi uniform) or totally molten (for uniform) and act as matrix material. Extrusion is an efficient way to melt a polysaccharide, such as described by Canadian patent No. 2,308,537 (Huppé et al) or Canadian patent No. 2,462,053 (Thibodeau et al) for example.

Regarding manufacturing techniques, operating with high pH solutions containing chromogenic compound, the addition of sodium hydroxide can cause the appearance of precipitated material that can decrease the homogeneity of the solution. The use of an alcohol can alleviate this issue, but for certain operating conditions and processes it is preferable to avoid using alcohols. Thus, the extrusion was preferably done with a solution containing no alcohol and prepared to have acidic pH between about 1.5 and about 7. When TMB chromogenic indicator was used, it dictated the pH of the solution since it is quite acidic. However, depending on the particular components of the composite material, different pHs and processing conditions may be appropriate.

"Peroxidatic activity" refers to the ability of catalytic substances to drive the reaction of hydroperoxides with colorless chromogenic electron donors which become fluorescent or visibly colored after oxidation.

"Pseudoperoxidatic activity" refers to the ability of an endogenous peroxidase or a non-peroxidase catalytic substance to drive the reaction of hydroperoxidases with colorless chromogenic electron donors which become fluorescent or visibly colored after oxidation. Certain transition metals and their ions and hemoproteins are known to have pseudoperoxidatic activity. Basophils, neutrophils, eosinophils and mast cells synthesize endogenous peroxidase which can be visualized at the ultrastructural level in the secretory apparatus of immature cells. Red blood cells and hematin containing compounds have iron as part of their heme groups, which can catalyze the oxidation of chromogenic electron donors. This pseudoperoxidatic activity can be inhibited with strong H₂O₂ solutions, sodium azide and methanol-H₂O₂ solutions.

"Peroxidatic assay" refers to any test or procedure that is based on a peroxidatic activity, as defined above, to generate a signal which can be detected or measured to evidence the presence of analyte or the amount present.

"Spontaneous oxidation" refers to the spontaneous production of visible color of a chromogenic electron donor in the absence of peroxidase enzyme.

"Chromogenic electron donor" refers to a compound which undergoes an easily observed change in color upon oxidation by an oxidizing agent such as a hydroperoxide. Examples of these are: benzidine, 3,3'-dimethylbenzidine (o-tolidine, OTD), 3,3'-dimethoxybenzidine (o-dianisidine, oDAD), 3,3'-diaminobenzidine (DAB), 3,3',5,5'-tetramethylbenzidine (TMB), 3,3',5,5' tetramethylbenzidinedihydrochloride, 3,3'-diethylbenzidine, 2,7-diaminofluorene (DAF), o-phenylenediamine (OPD), N,N-diethylphenylenediamine (DEPDA), N,N-dimethylphenylenediamine (DMPDA), 2,2'-azino-di (3-ethyl-benzthiazoline sulfonate) (ABTS), 3-methyl-2-benzothiazolinone hydrazone hydrochloride (MBTH), aminoethyl carbazole (AEC), and 4-chloro-1-naphthol (4-CN). Chromogenic compounds that are soluble in water, such as TMB hydrochloride, may be preferred.

"Hydroperoxide" refers to compounds of the general formula, ROOH, wherein the R group is an aryl, alkyl, or acyl group (organic hydroperoxide), or hydrogen atom (hydrogen peroxide).

"Storage lifetime" of a substrate-chromogen particles is the time interval after initial preparation of the indicator particles in which (a) the absorbance of said particles in the 400-800 nm wavelength range remains below 0.05 and (b) the peroxidatic activity of said solution is at least about 80% of its initial value, which corresponds to the stability limit by certain analyses.

"Nanocomposite(s)" refers to materials comprising a nanoscale dispersion of phyllosilicates in a polymer network. Typical phyllosilicate nanocomposites are exfoliated nanocomposites, intercalated nanocomposites and semi-exfoliated nanocomposites.

"Phyllosilicates" or "sheet-like silicates", in general refers to materials with parallel slicate-based sheets. Phyllosilicates can be aluminosilicates having a thickness of about 1 nm, while having a length and a width ranging from 300 to 500 nm. Phyllosilicates are a major constituent of clays. Size, composition and shape of phyllosilicates will vary depending on the clay sources. Phyllosilicates typically have the general molecular formula: Al2O.sub.3.4 SiO2.H.sub.2O.

"Intercalated nanocomposites", also referred to as "sandwich nanocomposites", refers to nanocomposites composed of repeating and alternating phyllosilicate-polymer layers. Within theses intercalated nanocomposites, the spacing between the phyllosilicate layers is increased to provide for the insertion of a polymer.

"Exfoliated nanocomposites", also referred to as "phyllosilicate dispersions", refers to nanocomposites comprising delaminated phyllosilicates which are dispersed throughout a polymeric network.

"Semi-exfoliated nanocomposites", refers to nanocomposites comprising partially exfoliated clays. Within these semi-exfoliated nanocomposites, clays are delaminated into smaller units comprising from about 45 to 70 phyllosilicate blocks. Clays usually comprise units having from about 85 to 140 phyllosilicate blocks as defined by Chenu et al. (Comptes Rendus de l'Acadmie des Sciences, Srie 2, 1990,310 (7 srie 2), PP. 975-980). Within these semi-exfoliated nanocomposites, the smaller phyllosilicate units are dispersed uniformly throughout the polymer.

In the context of certain embodiments of the composite material, intercalation is a preliminary step to the exfoliation of the clay. During manufacture of some embodiments of the composite material, the clay is mostly but not completely exfoliated. The degree of exfoliation will depend on the particular process and the process conditions that are used.

"Polysaccharide" refers to polymers comprising a backbone comprising at least 90% of monosaccharide repeating units and/or derivatized monosaccharide repeating units. Non-limiting examples include starches, modified starches, amylopectin, modified amylopectin, amylose, modified amylose, chitosan, chitin, guar gum, modified guar gum, locust bean gum, tara gum, konjac gum, konjac flour, fenugreek gum, mesquite gum, aloe mannans, cellulose, modified cellulose (representative examples include carboxyalkylated cellulose and carboxymethyl cellulose), oxidized polysaccharides, sulfated polysaccharides, cationic polysaccharides, pectin, arabic gum, karaya gum, xanthan, kappa, iota or lambda carrageenans, agar-agar and alginates. Non-limiting examples of mannose-based polysaccharides include guar gum, tara gum, locust bean gum, konjac, mesquite gum, and fenugreek extracts.

In addition, various organic and inorganic components described and referred to herein may be used to produce different embodiments of the composite material. It should also be noted that the components of embodiments of the composite material may be used in various proportions and concentrations. Though some particular exemplary concentrations are described in the examples, it should be noted that the components may optionally be used in concentrations in the range of ± 5 wt% with respect to the exemplary concentrations, and preferably in the range of ± 2 wt% with respect to the exemplary concentrations. In addition, exfoliated bentonite may be preferably used in a sufficiently low concentration and under conditions so as to avoid forming a gelled material that is non-fluid and thus difficult to introduce into the polysaccharide mixture, for instance about 0.5 wt% bentonite with respect to the total mixture.

### EXAMPLES

### Example 1

Several "recipes" are known and used for detecting blood in urine or faeces. However, the substrate that receives the chemically reactive agents also plays a significant role. In fact, it was found that certain solution compositions that were impregnated into paper supports (e.g. Whatman n° 4) resulting in paper-based indicators giving a sufficient color change response, were not effective solutions when applied substrates containing bentonite or other minerals. Such bentonite-containing substrates are notably less neutral than paper substrates, requiring adjustment of the composition of solutions to be dispersed into the bentonite-containing solutions.

Various solutions used in the following examples were developed and selected. It should be understood that these solutions are exemplary tests which were used in connection with an extrusion preparation method, and should not be seen as limiting.

### Example 2

Equipment: Coperion ^{™} twin screw extruder, model KSK25 WLE ^{™}, with 25 mm diameter, UD = 40, 10 barrels.
Extrusion conditions:
Extruder temperature profile

| | T °C |
|---|---|
| Barrel 2-3 | 34 |
| Barrel 4 | 110 |
| Barrel 5-6 | 110 |
| Barrel 7-8 | 40 |
| Barrel 9-10 | 40 |

Screw speed was 125 RPM.

### Products:

Corn starch C* Gel 03420™ introduced into the extruder. Water was injected at the second barrel on a basis of 21.3% relative to the total mass.

The following solution was prepared and introduced at the ninth barrel:

### Aqueous solution

| Product | TMB Hydrochloride (HC) | Cumene hydroperoxide | Lepidine | Citric acid | NaOH | pH |
|---|---|---|---|---|---|---|
| % | 0,41 | 0,32 | 0,12 | 4,48 | 1,48 | 4,2 |

The extruded mixture is dried at 50 °C. The product already absorbs one time its own weight of liquid after five contact seconds, which ensures effective urine absorption in short timeframe, facilitating contact with the reactive agents. In addition, the studies show that the product can absorb large amounts of liquid, approximately four times its own weight over an hour of contact time, which enables intense and durable coloration of the pellets and prolonged reactivity in case multiple urine applications occur. See Fig 3 showing absorption versus time for different types of pellets.

In the presence of urine containing haemoglobin, a light bluish localised coloration appeared.

### Example 3

The same installation as in Example 2 was used. The temperature profile in the extruder was the following:
Extruder temperature profile

| | T °C |
|---|---|
| Barrel 2-3 | 34 |
| Barrel 4 | 110 |
| Barrel 5-6 | 110 |
| Barrel 7-8 | 40 |
| Barrel 9-10 | 40 |

Corn starch C* Gel 03420™ (Cargill™) was used.

The following solution was prepared:
Aqueous solution

| Product | TMB HC | Cumene HP | Lepidine | Citric acid | NaOH | Ascorbic Acid | Tensio-active | pH |
|---|---|---|---|---|---|---|---|---|
| % | 0,33 | 0,26 | 0,18 | 3,61 | 1,19 | 0,03 | 0,41 | 4,7 |

The same operating conditions as in Example 2 were used. The product that was obtained was clear yellow and reacted when put in contact with blood.

### Example 4

The same installation as in Example 2 was used. The temperature profile in the extruder was the following:
Extruder temperature profile

| | T °C |
|---|---|
| Barrel 2-3 | 35 |
| Barrel 4 | 110 |
| Barrel 5-6 | 110 |
| Barrel 7-8 | 41 |
| Barrel 9-10 | 38 |

Wheat starch, Whetstar 4^{™} (ADM^{™}) was used. Exfoliated bentonite 0.5%, WT-325 mesh National Premium (BPN) was also used. The water "solvent" for the bentonite was 20.8% of the total weight. The chromogenic solution was the same as that of Example 3 and was introduced at barrel 9.

The product that was obtained was much more reactive. However, it has a certain heterogeneity due to the short mixing time in barrels 9 and 10 prior to the extruder outlet. An intense blue-green develops rapidly when in contact with blood, which is in sharp contrast with its initial light amber color prior to reaction.

### Example 5

Following the extrusion installation of Example 4, a mono-screw extrruder was introduced (mut^{™}, model Breba^{™}). The screw diameter was 48 mm, UD = 20/1 to increase the mixing and enable improved homogenisation of the product.

The temperature profile in the extruder was the following:
Extruder temperature profile

| | T °C |
|---|---|
| Barrel 2-3 | 35 |
| Barrel 4 | 110 |
| Barrel 5-6 | 110 |
| Barrel 7-8 | 41 |
| Barrel 9-10 | 38 |

Wheat starch, Whetstar 4^{™} (ADM^{™}) was used. The aqueous solution was the same as that used in Example 2. Improvement in homogenisation and dispersion of the components was achieved.

### Example 6

Another example was done where the reactive components were introduced separately into the polysaccharide mixture. At the inlet of the extruder, for each 1 kg of starch, a 200 g aqueous solution containing 0.46 g of TMB-HC, 0.24 g lepidine and 0.5 g bentonite, were used. Near the outlet of the extruder, another solution (60 g) was introduced containing 0.12 g EDTA, 5 g citric acid and 0.36 g cumene hydroperoxide. The same temperature profile was used as for the previous example.

### Example 7

For the purpose of comparison, some solution compositions, which may function as desired when impregnated into paper substrates, provided less effective activity when combined with the starch-bentonite mixture by extrusion:

| Solution A | Solution B | Solution C | Product % |
|---|---|---|---|
| 0,35 | 0,35 | 0,54 | TMB |
| 0,27 | 0,27 | 0,42 | C HP |
| 0,18 | 0,18 | 0,18 | Lepidine |
| 3,8 | 3,8 | 3,8 | Citric acid |
| 1 | 1,27 | 1,27 | NAOH |

The concentrations and relative proportions used in the other examples enabled composite materials with superior processability and color indication properties than those prepared using solutions A-C.

The present specification refers to a number of documents, the contents of which are incorporated herein by reference.

The present invention has been described with regard to preferred embodiments. The description and the drawings were intended to help the understanding of the invention and should not be used to unduly limit its scope. It will be apparent to one skilled in the art that various modifications may be made to the invention without departing from the scope of what has actually been invented.

## Claims

1. A composite litter material for detecting a disease when contacted by feline urine, the composite litter material comprising:
an absorptive polymer material forming a solid matrix;
exfoliated clay embedded and dispersed within the solid matrix;
a chromogenic indicator provided within the solid matrix; and
an oxidizing agent distributed and stabilized within the solid matrix such that the oxidizing agent is available and responsive to peroxidase or
pseudoperoxidase activity in the urine to activate the chromogenic indicator.

2. The composite material of claim 1, wherein the absorptive polymer material is a polysaccharide, optionally comprising alpha-linkages.

3. The composite material of claim 1, wherein the exfoliated clay comprises exfoliated phyllosilicates, which are optionally dispersed throughout the entire solid matrix.

4. The composite material of claim 1, wherein the chromogenic indicator is cationic and the exfoliated clay comprises sheets having anionic surface characteristics.

5. The composite material of claim 1, wherein the chromogenic indicator is a benzidine-based compound, optionally corresponding to the following formula: 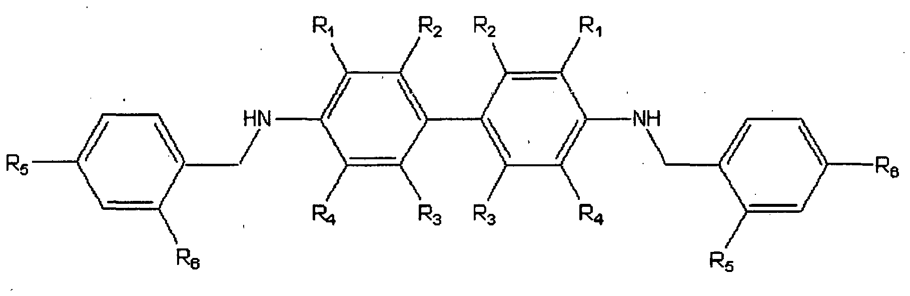 wherein R1, R2, R3 and R4 are the same or different and represent hydrogen, halogen, a lower alkyl or alkoxy group containing 1 to 4 carbon atoms, a (C1 -C4)-dialkylamino group, an acetylamino group, a nitro group or an aromatic group which may be substituted; and
wherein R5 and R6 are the same or different and represent water-soluble groups, hydroxyl group, amino group, acidic group, disulfonyl group, ether group, halogen, and a lower alkyl or alkoxy group containing 1 to 4 carbon atoms, a (C1 -C4)-dialkylamino group, an acetylamino group or a nitro group.

6. The composite material of claim 1, wherein the chromogenic indicator is 3,3',5,5'-tetramethylbenzidine (TMB) or 3,3',5,5' tetramethylbenzidine dihydrochloride (TMB-HC).

7. The composite material of claim 1, wherein the oxidizing agent is a hydroperoxide, optionally cumene hydroperoxide or diisopropylbenzene dihydroperoxide

8. The composite material of claim 7, wherein the absorptive polymer material comprises polyols having polymer backbones and the hydroperoxide is stabilized along the polymer backbones.

9. The composite material of claim 1, having the form of discrete pellets, optionally having a disk shape, a lozenge shape, a wafer shape or a cat head shape, and optionally having a diameter between 1 mm to 10 mm and a thickness between 0.5 mm and 3 mm.

10. The composite material of claim 1, further comprising an impregnated stabilizer composition, optionally comprising a color enhancer.

11. A method of manufacturing a composite animal litter material according to claim 1 for detecting a feline disease, comprising:
preparing a mixture comprising an absorptive polymer and water;
distributing exfoliated clay, a chromogenic indicator and an oxidizing agent to within the mixture to produce the composite animal litter material.

12. The method of claim 11, wherein a solution comprising the exfoliated clay is added to the mixture during the preparation thereof and wherein a chromogenic solution comprising the chromogenic indicator, a solvent and the oxidizing agent is prepared and introduced into the mixture.

13. The method of claim 12, wherein the chromogenic solution and the mixture are combined by extrusion, pressure agglomeration, tumble growth agglomeration or matrix melt formation, or a combination thereof.

14. The method of claim 13, wherein the chromogenic solution is buffered at a pH between 1.5 and 7, optionally buffered at a pH allowing solubility in the absence of alcohol.

15. The method of claim 14, wherein the chromogenic solution and the mixture are combined by extrusion.

16. The method of claim 15, wherein the extrusion further comprises:
providing an extrusion apparatus having an upstream section, a mid section and a downstream section;
feeding the mixture into the upstream section of the extrusion apparatus;
feeding the chromogenic solution into the mixture at the downstream section of the extrusion apparatus, to form a combined blend;
extruding, cutting and drying the combined blend to form the composite material.

17. The method of claim 16, wherein the mid section is operated at a higher temperature than the upstream and downstream sections, optionally wherein the temperature of the mid section is 60 to 80°C higher than either one of the upstream or downstream sections, or optionally wherein the upstream section is operated at a temperature between 25 °C and 40°C, the mid section is operated at a temperature between 80°C and 130°C, and the downstream section is operated at a temperature between 25 °C and 40°C, or optionally wherein the upstream section is operated at a temperature of 30°C, the mid section is operated at a temperature of 110 °C, and the downstream section is operated at a temperature of 40°C.

18. The method of claim 16, wherein the extrusion apparatus comprises a twin screw extruder, optionally comprising a first extruder having an inlet and an outlet, and a second extruder coupled to the first extruder to receive the combined blend from the outlet of the first extruder, wherein the first extruder optionally is a twin-screw extruder or a single screw extruder.

## Patentansprüche

1. Verbundstreumaterial zum Detektieren einer Krankheit, wenn es mit Katzenurin in Kontakt kommt, wobei das Verbundstreumaterial umfasst:
ein absorptionsfähiges Polymermaterial, das eine feste Matrix bildet;
abgeblätterten Ton, der in der festen Matrix eingebettet und dispergiert ist;
einen chromogenen Indikator, der in der festen Matrix angeordnet ist, und
ein Oxidationsmittel, das in der festen Matrix verteilt und stabilisiert ist, so dass das Oxidationsmittel für/auf Peroxidase oder Pseudoperoxidaseaktivität im Urin verfügbar und ansprechend ist, um den chromogenen Indikator zu aktivieren.

2. Verbundmaterial nach Anspruch 1, wobei das absorptionsfähige Polymermaterial ein Polysaccharid ist, das gegebenenfalls alpha-Verknüpfungen umfasst.

3. Verbundmaterial nach Anspruch 1, wobei der abgeblätterte Ton abgeblätterte Phyllosilicate umfasst, die gegebenenfalls durch die gesamte feste Matrix verteilt sind.

4. Verbundmaterial nach Anspruch 1, wobei der chromogene Indikator kationisch ist und der abgeblätterte Ton Blätter umfasst, die anionische Oberflächencharakteristika haben.

5. Verbundmaterial nach Anspruch 1, wobei der chromogene Indikator eine Benzidin-basierte Verbindung ist, die gegebenenfalls der folgenden Formel entspricht: 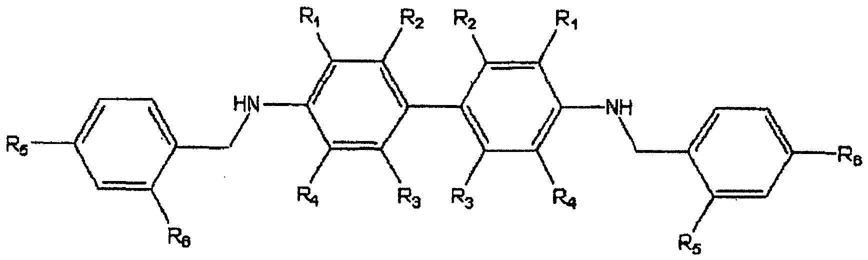 worin R₁, R₂, R₃ und R₄ gleich oder unterschiedlich sind und Wasserstoff, Halogen, eine Niederalkyl- oder -alkoxygruppe, die 1 bis 4 Kohlenstoffatome enthält, eine (C1-C4)-Dialkylaminogruppe, eine Acetylaminogruppe, eine Nitrogruppe oder eine aromatische Gruppe, die substituiert sein kann, darstellen und
worin R₅ und R₆ gleich oder unterschiedlich sind und wasserlösliche Gruppen, Hydroxylgruppe, Aminogruppe, saure Gruppe, Disulfonylgruppe, Ethergruppe, Halogen und eine Niederalkyl oder -alkoxygruppe, die 1 bis 4 Kohlenstoffatome enthält, eine (C1-C4)-Dialkylaminogruppe, eine Acetylaminogruppe oder eine Nitrogruppe darstellen.

6. Verbundmaterial nach Anspruch 1, wobei der chromogene Indikator 3,3',5,5'-Tetramethylbenzidin (TMB) oder 3,3',5,5'-Tetramethylbenzidin-Dihydrochlorid (TMB-HC) ist.

7. Verbundmaterial nach Anspruch 1, wobei das Oxidationsmittel ein Hydroperoxid, gegebenenfalls Cumenhydroperoxid oder Diisopropylbenzoldihydroperoxid, ist.

8. Verbundmaterial nach Anspruch 7, wobei das absorptionsfähige Polymermaterial Polyole, die Polymerhauptketten haben, umfasst und das Hydroperoxid entlang der Polymerhauptketten stabilisiert ist.

9. Verbundmaterial nach Anspruch 1, das die Form von getrennten Pellets hat, die gegebenenfalls eine Scheibenform, eine Zungenform, eine Waffelform oder eine Katzenkopfform haben und die gegebenenfalls einen Durchmesser zwischen 1 mm und 10 mm und eine Dicke zwischen 0,5 mm und 3 mm haben.

10. Verbundmaterial nach Anspruch 1, das außerdem eine imprägnierte Stabilisatorzusammensetzung umfasst, die gegebenenfalls einen Farbverstärker umfasst.

11. Verfahren zur Herstellung eines Verbundtierstreumaterials nach Anspruch 1 zum Detektieren einer Katzenkrankheit, umfassend:
Herstellen eines Gemisches, das ein absorptionsfähiges Polymer und Wasser umfasst;
Verteilen von abgeblättertem Ton, einem chromogenen Indikator und einem Oxidationsmittel in dem Gemisch, um das Verbundtierstreumaterial herzustellen.

12. Verfahren nach Anspruch 11, wobei eine Lösung, die den abgeblätterten Ton umfasst, zu dem Gemisch während der Herstellung desselben gegeben wird und wobei eine chromogene Lösung, die den chromogenen Indikator, ein Lösungsmittel und das Oxidationsmittel umfasst, hergestellt wird und in das Gemisch eingeführt wird.

13. Verfahren nach Anspruch 12, wobei die chromogene Lösung und das Gemisch durch Extrusion, Druckagglomeration, Trommelwachstumsagglomeration oder Matrixschmelzbildung oder eine Kombination davon kombiniert werden.

14. Verfahren nach Anspruch 13, wobei die chromogene Lösung bei einem pH zwischen 1,5 und 7 gepuffert ist, gegebenenfalls bei einem pH gepuffert ist, der Löslichkeit in Abwesenheit von Alkohol erlaubt.

15. Verfahren nach Anspruch 14, wobei die chromogene Lösung und das Gemisch durch Extrusion kombiniert werden.

16. Verfahren nach Anspruch 15, wobei die Extrusion außerdem umfasst:
Bereitstellen eines Extruders, der einen stromaufwärts gelegenen Abschnitt, einen mittleren Abschnitt und einen stromabwärts gelegenen Abschnitt hat;
Einspeisen des Gemisches in den stromaufwärts gelegenen Abschnitt des Extruders;
Einspeisen der chromogenen Lösung in das Gemisch im stromabwärts gelegenen Abschnitt des Extruders unter Bildung einer kombinierten Mischung;
Extrudieren, Schneiden und Trocknen der kombinierten Mischung unter Bildung des Verbundmaterials.

17. Verfahren nach Anspruch 16, wobei der mittlere Abschnitt bei einer höheren Temperatur als der stromaufwärts gelegene und der stromabwärts gelegene Abschnitt betrieben wird, wobei gegebenenfalls die Temperatur des mittleren Abschnitts 60 bis 80°C höher ist als die eines der stromaufwärts gelegenen oder stromabwärts gelegenen Abschnitte, oder wobei gegebenenfalls der stromaufwärts gelegene Abschnitt bei einer Temperatur zwischen 25°C und 40°C betrieben wird, der mittlere Abschnitt bei einer Temperatur zwischen 80°C und 130°C betrieben wird und der stromabwärts gelegene Abschnitt bei einer Temperatur zwischen 25°C und 40°C betrieben wird, oder wobei gegebenenfalls der stromaufwärts gelegene Abschnitt bei einer Temperatur von 30°C betrieben wird, der mittlere Abschnitt bei einer Temperatur von 110°C betrieben wird und der stromabwärts gelegene Abschnitt bei einer Temperatur von 40°C betrieben wird.

18. Verfahren nach Anspruch 16, wobei der Extruder einen Doppelschneckenextruder umfasst, der gegebenenfalls einen ersten Extruder, der eine Einlassöffnung und eine Auslassöffnung hat, und einen zweiten Extruder, der an den ersten Extruder gekoppelt ist, um die kombinierte Mischung aus der Auslassöffnung des ersten Extruders aufzunehmen, umfasst, wobei der erste Extruder gegebenenfalls ein Doppelschneckenextruder oder ein Einzelschneckenextruder ist.

## Revendications

1. Matériau composite de litière permettant de détecter une maladie lors d'un contact avec de l'urine de félin, le matériau composite de litière comprenant :
un matériau polymère absorbant formant une matrice solide ;
de l'argile exfoliée incorporée et dispersée au sein de la matrice solide ;
un indicateur chromogène prévu au sein de la matrice solide ; et
un agent oxydant distribué et stabilisé au sein de la matrice solide de sorte que l'agent oxydant est disponible et réactif à une activité de peroxydase ou de pseudo-peroxydase dans l'urine afin d'activer l'indicateur chromogène.

2. Matériau composite selon la revendication 1, dans lequel le matériau polymère absorbant est un polysaccharide, comprenant facultativement des liaisons alpha.

3. Matériau composite selon la revendication 1, dans lequel l'argile exfoliée comprend des phyllosillicates exfoliés, qui sont facultativement dispersés à travers la totalité de la matrice solide.

4. Matériau composite selon la revendication 1, dans lequel l'indicateur chromogène est cationique et l'argile exfoliée comprend des feuilles ayant des caractéristiques de surface anioniques.

5. Matériau composite selon la revendication 1, dans lequel l'indicateur chromogène est un composé à base de benzidine, correspondant facultativement à la formule suivante : 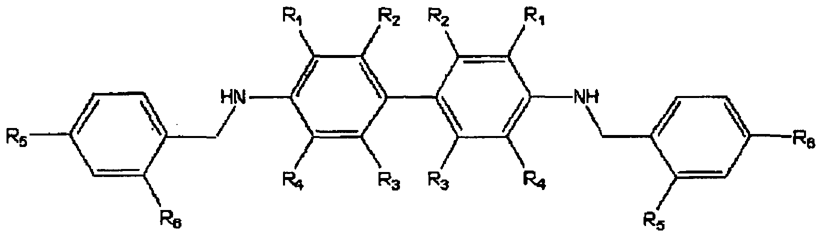 dans laquelle R₁, R₂, R₃ et R₄ sont identiques ou différents et représentent un hydrogène, un halogène, un groupe alkyle ou alcoxy inférieur contenant de 1 à 4 atomes de carbone, un groupe dialkylamino (en C₁ à C₄), un groupe acétylamino, un groupe nitro ou un groupe aromatique qui peut être substitué ; et
dans laquelle R₅ et R₆ sont identiques ou différents et représentent des groupes solubles dans l'eau, un groupe hydroxyle, un groupe amino, un groupe acide, un groupe disulfonyle, un groupe éther, un halogène, et un groupe alkyle ou alcoxy inférieur contenant de 1 à 4 atomes de carbone, un groupe dialkylamino (en C₁ à C₄) , un groupe acétylamino ou un groupe nitro.

6. Matériau composite selon la revendication 1, dans lequel l'indicateur chromogène est la 3,3',5,5'-tétraméthylbenzidine (TMB) ou le dichlorhydrate de 3,3',5,5'-tétraméthylbenzidine (TMB-HC)

7. Matériau composite selon la revendication 1, dans lequel l'agent oxydant est un hydroperoxyde, facultativement l'hydroperoxyde de cumène ou le dihydroperoxyde de diisopropylbenzène.

8. Matériau composite selon la revendication 7, dans lequel le matériau polymère absorbant comprend des polyols ayant des squelettes polymères et l'hydroperoxyde est stabilisé le long des squelettes polymères.

9. Matériau composite selon la revendication 1, ayant la forme de granulés distincts, ayant facultativement une forme de disque, une forme de losange, une forme de pastille ou une forme de tête de chat, et ayant facultativement un diamètre entre 1 mm à 10 mm et une épaisseur entre 0,5 mm et 3 mm.

10. Matériau composite selon la revendication 1, comprenant en outre une composition de stabilisant imprégnée, comprenant facultativement un renforçateur de couleur.

11. Procédé de fabrication d'un matériau composite de litière animale selon la revendication 1, permettant de détecter une maladie féline, comprenant :
la préparation d'un mélange comprenant un polymère absorbant et de l'eau ;
la distribution d'une argile exfoliée, d'un indicateur chromogène et d'un agent oxydant au sein du mélange afin de produire le matériau composite de litière animale.

12. Procédé selon la revendication 11, dans lequel une solution comprenant l'argile exfoliée est ajoutée au mélange pendant sa préparation et dans lequel une solution chromogène comprenant l'indicateur chromogène, un solvant et l'agent oxydant est préparée et introduite dans le mélange.

13. Procédé selon la revendication 12, dans lequel la solution chromogène et le mélange sont combinés par extrusion, agglomération par pression, agglomération de croissance par agitation ou formation à l'état fondu de matrice, ou une combinaison de celles-ci.

14. Procédé selon la revendication 13, dans lequel la solution chromogène est tamponnée à un pH entre 1,5 et 7, facultativement tamponnée à un pH permettant une solubilité en l'absence d'alcool.

15. Procédé selon la revendication 14, dans lequel la solution chromogène et le mélange sont combinés par extrusion.

16. Procédé selon la revendication 15, dans lequel l'extrusion comprend en outre :
la fourniture d'un appareil d'extrusion ayant une section amont, une section milieu et une section aval ;
l'apport du mélange dans la section amont de l'appareil d'extrusion ;
l'apport de la solution chromogène dans le mélange au niveau de la section aval de l'appareil d'extrusion, afin de former un mélange combiné ;
l'extrusion, la découpe et le séchage du mélange combiné afin de former le matériau composite.

17. Procédé selon la revendication 16, dans lequel la section du milieu est exploitée à une température supérieure aux sections amont et aval, facultativement dans lequel la température de la section du milieu est plus élevée de 60 à 80 °C que l'une ou l'autre des sections amont ou aval, ou facultativement dans lequel la section amont est exploitée à une température entre 25 °C et 40 °C, la section du milieu est exploitée à une température entre 80 °C et 130 °C, et la section aval est exploitée à une température entre 25 °C et 40 °C, ou facultativement dans lequel la section amont est exploitée à une température de 30 °C, la section du milieu est exploitée à une température de 110 °C et la section aval est exploitée à une température de 40 °C.

18. Procédé selon la revendication 16, dans lequel l'appareil d'extrusion comprend une extrudeuse à double vis, comprenant facultativement une première extrudeuse ayant une admission et un refoulement, et une seconde extrudeuse couplée à la première extrudeuse afin de recevoir le mélange combiné depuis le refoulement de la première extrudeuse, dans lequel la première extrudeuse est facultativement une extrudeuse à double vis ou une extrudeuse à simple vis.
